Europäisches Patentamt

European Patent Office

Office européen des brevets

(19)

(11) EP 1 577 315 A1

(12) EUROPEAN PATENT APPLICATION
published in accordance with Art. 158(3) EPC

(43) Date of publication:
21.09.2005 Bulletin 2005/38

(51) Int Cl.⁷: C07G 17/00

(21) Application number: 03778886.6

(86) International application number:
PCT/JP2003/015959

(22) Date of filing: 12.12.2003

(87) International publication number:
WO 2004/052898 (24.06.2004 Gazette 2004/26)

(84) Designated Contracting States:
AT BE BG CH CY CZ DE DK EE ES FI FR GB GR
IE IT LI LU MC NL PT SE SK TR
Designated Extension States:
AL LT LV MK

(30) Priority: 12.12.2002 JP 2002360424

(71) Applicant: Asahi Breweries, Ltd.
Tokyo 104-0031 (JP)

(72) Inventors:
• TAGASHIRA, Motoyuki, Asahi Breweries, Ltd.
Moriya-shi, Ibaraki 302-0106 (JP)
• KANDA, Tomomasa, Asahi Breweries, Ltd.
Moriya-shi, Ibaraki 302-0106 (JP)

(74) Representative: Ziebig, Marlene et al
Anwaltskanzlei
Gulde Hengelhaupt Ziebig & Schneider
Wallstrasse 58/59
10179 Berlin (DE)

(54) PROCESS FOR PRODUCING HOP GLUME POLYPHENOL

(57) It is intended to provide a process for efficiently producing highly purified hop bract polyphenol using hop bract as the starting material, and foods, drinks, cosmetics and drugs with the use of the highly purified hop bract polyphenol. Namely, a process for producing hop polyphenol which comprises extracting hop bract with an aqueous alcohol solution, concentrating the extract to give a residual alcohol concentration of 0.5 to 2% and then centrifuging and/or filtering the concentrate. This polyphenol is usable in foods, drinks, cosmetics, drugs, etc.

Fig.1

EP 1 577 315 A1

**Description**

Technical Field

This invention relates to a process for producing hop bract polyphenol and the use.

Background Art

Hop (*Humulus lupulus*) is a parennial plant of a Cannabaceae family, and the hop cone (a ripe unfertilized mature female flower) is generally called hop. The hop comprises, in addition to the cones, leaves, bines, roots and the like. A lupulin part (a yellow granule formed in the root of internal bracts of the hop cone) existing in the hop cone is a source of bitterness and perfume, and it is an important beer material along with yeast and malt in beer brewing. The hop can be used as a sedative drug and an anti-aphrodisiac in folk remedies. The hop bracts are formed by removing the lupulin part from the hop cone, and these bracts are useless. If circumstances require, these bracts are removed in beer brewing to be byproducts. In this case, the hop bracts are used as fertilizer or livestock forage. However, since effective use is not found, it is hoped to develop a method having a high additional value for using the bracts.

In the following Japanese Patent Laid-open Publications 1-6 of the applicant's application, it is recognized that hop, particularly the polyphenols derived from hop bracts have antioxidant action, sparkle-stabilizing action of sparkling malt drinks, anticaries action, deodorant action, antimetastatic action of cancer cells, and topoisomeraze-inhibiting action.

1. Japanese Patent Laid-open Publication No. 09-2917,
2. Japanese Patent Laid-open Publication No. 09-163969,
3. Japanese Patent Laid-open Publication No. 09-295944,
4. Japanese Patent Laid-open Publication No. 10-25232,
5. Japanese Patent Laid-open Publication No. 2000-327582,
6. Japanese Patent Laid-open Publication No. 2001-39886,

Various uses of hop bract polyphenol have been reported as described in the above. However, the hop bract polyphenol is not used industrially and positively. As the reason, working and process controls in the production of hop bract polyphenol are difficult, because impurity such as wax and fibers derived from the hop bract produce a large amount of precipitates.

Disclosure of Invention

The inventors of this invention earnestly have studied and found a process comprising extracting hop bract with an aqueous alcohol solution, concentrating the extract to give a residual alcohol concentration of 2 or less v/v%, purifying the concentrate by centrifuging or filtering, and easily obtaining the hop bract polyphenol having high purity.

Hitherto, some methods for producing the hop bract polyphenol have been disclosed in the above patent references 1-6. However, these references do not disclose the purification process that hop bract is extracted with an aqueous alcohol solution, the extract is concentrated into 2 v/v% or less of remaining alcohol concentration, the concentrate is purified by centrifugation and/or filtering. The inventors have completed this invention by using the resulting substance for food and drink, beauty aids, medicines and the like. As the medicines, an anti-inflammatory agent, an anti-allergy agent, an agent for inhibition of dental plaque formation, an anticancer agent, and an antimetastatic agent of cancer cells can be provided.

Brief Description of Drawings

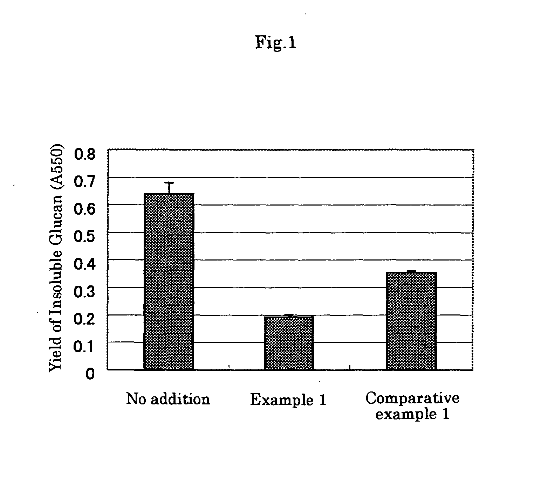

Fig. 1 is a graph showing difference of inhibition activities of insoluble glucan-producing enzymes between the hop bract polyphenol prepared by this invention (Example 1) and the hop bract polyphenol prepared by conventional method (Comparison example 1). In the figure, compared with control (no addition), lower column shows that insoluble glucan-producing enzymes are inhibited and the producing amount of insoluble glucan is less.

Best Mode for Carrying out the Invention

The hop bract of the raw materials of this invention is obtained by removing the lupulin part from the hop cone. Generally, the hop bract is obtained by crushing the hop cone and filtering the hop bract to remove the lupulin part. After removing the lupulin part, the hop bract may be used for raw materials as it is, or after processing the hop bract into a pellet form for transportation, it may be used. On the other hand, raw materials containing the hop bract such as hop cone itself or the residue of supercritical fluid extraction may be used as the raw materials without any problems.

The method for producing the hop bract polyphenol comprises extracting hop bract or hop bract pellet of the raw materials with an aqueous alcohol solution of 50 v/v% or less. As a preferred example, an aqueous alcohol solution is an aqueous ethanol solution of 50 v/v% or less. The ratio of the raw material and extracted solvent is preferably about 1:10-20 (weight ratio), the extraction is conducted at a temperature of 30-60°C with stirring for 60-180 minutes. The extract is obtained by filtering, if necessary, the filtration is done by compressing with belting press and the like to increase the recovery of the extract. In addition, water is added again to the compressed hop bract, and the mixture is compressed again to increase the recovery of the extract.

The resulting hop bract extract is a green to whity green liquid. Then, the liquid is concentrated into a residual alcohol concentration of 2 v/v% or less and purified. If the concentrate is not purified and it is treated in the next process, since a large amount of precipitates derived from wax and fibers are produced, serious troubles are caused in working.

When the hop bract extract is concentrated into a residual alcohol concentration of 2 v/v% or less, the extract is concentrated by a common method such as heat concentration or vacuum concentration without any problems. In the process, alcohol may be recovered and reused.

The purification process is conducted by centrifuging or filtering the hop bract extract which is concentrated into a residual alcohol concentration of 2 v/v% or less. In the centrifuging process, a common centrifuge may be a continuous type or a centrifuge tube type without any problems. A continuous type centrifuge having centrifugation at about 50-10,000G may be preferably used.

If necessary, the hop bract extract may be maintained at a temperature of 10°C or less from several days to about 1 month. The precipitates are developed well enough and removed.

The raw material solutions obtained by the above purifying process are treated by an adsorption process that the hop bract polyphenol is adsorbed to a synthetic adsorbent of a gel type, a washing process that the synthetic adsorbent is washed with water or an aqueous ethanol solution, preferably, water or an aqueous ethanol solution of 10 v/v% or less, and an elution process that the adsorption fraction is eluted from the synthetic adsorbent with an aqueous ethanol solution of more than 30 v/v% or ethanol to obtain the hop bract polyphenol.

In the adsorption process, the extract was cooled to a room temperature of 15-30°C, and passed through a column filled with the synthetic adsorbent to adsorb hop bract polyphenol into the adsorbent. The synthetic adsorbent of a gel type is a hydrophilic vinyl polymer, hydroxyl propylated dextran, a styrene-divinylbenzen polymer or the like. Liquid-passing time is preferably a SV value of 0.5-10. The SV value is defined by the following formula.

$$SV \text{ value} = (\text{Liquid-passing volume (L)}) / [(\text{resin volume (L)}) \times (\text{liquid-passing time(h)})]$$

In the washing process, the synthetic adsorbent of a gel type is washed to remove impurity components for increasing the purity of the hop bract polyphenol. The washing solvent is preferably water or an aqueous ethanol solution of 1-10 v/v%, and the volume is 1-5 times of the resin volume.

In the elution process, hop bract polyphenol is eliminated and eluted from the synthetic adsorbent of a gel type that keeps the hop bract polyphenol. The solvent for eluting may be hydrous alcohol, hydrous acetone, hydrous acetonitrile or the like, particularly an aqueous solution of more than 30 v/v% of ethanol or ethanol. The liquid-passing volume of the elution solvent is preferably 1-6 times of the resin volume.

The resulting eluate is concentrated under reduced pressure. The solvent is removed by a common method such as freeze dry or splay dry and powder hop bract polyphenol can be obtained. In the concentration under reduced pressure, solvent such as alcohol, acetone, acetonitrile or the like can be recovered to reuse. Such obtained hop bract polyphenol is faint bitter orderless powder of a skin color, brown or pale yellow.

The yield is 1-10 w/w% in hop bract weight of reduced weight. The used synthetic adsorbent of a gel type is washed with an aqueous alcohol solution, about 0.05 N-sodium hydroxide solution or the like, the adsorbent can be repeatedly used.

The resulting polyphenol can be mixed in necessary amount of each use such as for food and drink, beauty aids, quasi-drugs, medicines and the like. For example, when the hop bract polyphenol is used as an agent for inhibition of dental plaque formation, it is used in food and drinks such as confectionery, foodstuffs and beverages, particularly candy, chocolates, caramel, chewing gum and the like that can be retained in the mouth relatively long time. It may be added to medicines for oral cavity such as gargle, dentifrice, mouthwash and the like. When the hop bract polyphenol is added into these food and drinks and medicines for oral cavity, the hop polyphenol may be added as it is. Preferably, an aqueous solution, an aqueous alcohol solution or an alcohol solution of 1-2 v/v% hop bract polyphenol may be added to food and drinks or medicines for oral cavity at a final concentration of 10-5000ppm, preferably 100-1000ppm.

The hop bract polyphenol can be formulated along with a carrier, adjuvant, or additive of general use to utilize as an oral or parenteral pharmaceutical product in general methods. As the medicines, an anti-inflammatory agent, an anti-allergy agent, an agent for inhibition of dental plaque formation, an anticancer agent, and an antimetastatic agent of cancer cell can be provided.

Oral medical supplies are tablets, capsules, granule, syrup and the like, and parenteral medical supplies are external medicines such as ointment, cream and solution, and injectables such as sterility solutions and suspension. When these supplies are administered to human bodies, the administration amounts are 2mg-500mg per one or few times per day, 2mg-1,000mg per day to give sufficient effects.

The medical supplies containing the hop bract polyphenol of the present invention can be formulated by a desired unit volume form along with pharmaceutically approved vehicles, carriers, fillers, binders, stabilizers, flavors and the like. Adjuvants mixable with tablets or capsules are as follows; binders such as tragacanth, arabic gum, corn starch and geratin, fillers such as microcellulose, swelling agents such as corn starch, all geratinization starch, alginic acid, lubricants such as magnesium stearate, sweetening agents such as sucrose and saccharin, and flavors such as peppermint, akamono oil and cherries. The capsules can contain liquid carriers such as fats and fatty oils in addition to the above materials. The other materials are coating agents. Further, the physical forms of formulations can be changed by the other methods. As examples, tablets can be coated with shellac or sucrose. Syrup or elixir can contain sucrose as a sweetening agent, methyl or propylparaben as an antiseptic, a pigment and a flavor such as a cherry or orange flavor.

Sterilized constitution for injection can be formulated by common methods that active materials in vehicles such as water for injection, natural plant oils such as sesame oil, coconut oil, peanut oil and cotton seed oil, or synthetic fatty vehicles such as ethyl oleate are dissolved or suspended. Moreover, if necessary, buffer, antiseptic, antioxidant and the like can be formulated. External medicines such as ointment or cream can be obtained by common methods using Vaseline, paraffin, fats and fatty oils, lanolin, macrogol and the like.

The hop bract polyphenol of this invention may be added into food and drinks. The food and drinks containing the hop bract polyphenol may be formed by the above formulation. Otherwise, it may be processed by general methods by adding requirement into food materials of wheat gluten, Japanese cracker, cookies or drinks. The hop bract polyphenol is processed and orally taken as health foods or functionality foods of 5mg-500mg per day by dividing into a few times for ill prevention and health maintenance.

When the hop bract polyphenol is added to these food and drink, it is desired that the polyphenol may be added as powder, in the final concentration of 10-500ppm, and preferably, 10-100ppm in a 1-2% aqueous solution or aqueous alcohol solution or alcoholic solution.

Example

Although the following examples are described in detail the embodiment of this invention, this invention is not limited into these examples.

Example 1 (Preparation of the hop bract polyphenol by the purifying process)

Hop bract 50g was extracted with a 40v/v% ethanol solution 1 litter by stirring at a temperature of 40°C for 60 minutes. After compressed filtration with gauze, water was further added and recompressed and filtered to obtain extract 1 litter. This extract was concentrated to 500 ml with a rotary evaporator. The remaining ethanol concentration was about 1%. This concentrate was treated with a centrifuge at 4,000G for 15 minutes and filtered with filter paper. The extract was passed through a column of stylene-divinyl benzene resin (manufactured by Mitsubishi Kagaku Co., SP850) 30g by SV=1, and then the column was washed with water 100 ml. An aqueous 65v/v% ethanol solution 120ml was passed through the column, and the eluate 120 ml was recovered. This eluate was concentrated to 30 ml with the rotary evaporator. After freeze-dried, the hop bract polyphenol 1.7g was obtained as odorless brown powder having faint bitter taste.

Comparison example 1 (Preparation of the hop bract polyphenol by a conventional method)

Hop bract 50g was extracted with a 40v/v% ethanol solution 1 litter by stirring at a temperature of 40°C for 60 minutes. After compressed filtration with gauze, water was further added and recompressed and filtered to obtain extract 1 litter. This extract was concentrated to 500 ml with a rotary evaporator. The remaining ethanol concentration was about 1%. This concentrate was passed through a column of stylene-divinyl benzene resin (manufactured by Mitsubishi Kagaku Co., SP850) 30g by SV=1, and then the column was washed with water 100 ml. An aqueous 65v/v% ethanol solution 120ml was passed through the column, and the eluate 120ml was recovered. This eluate was concentrated to 30 ml with the rotary evaporator. After freeze-dried, the hop bract polyphenol 1.8g was obtained as odorless brown powder having faint bitter taste.

| Example 2 (dentifrice) | |
|---|---|
| Dibasic calcium phosphate | 42.0 |
| Glycerin | 18.0 |
| Carrageenan | 0.7 |
| Sodium lauryl sulfate | 1.2 |
| Sodium saccharin | 0.09 |
| Butyl p-hydroxy benzoate | 0.005 |
| Hop bract polyphenol obtained as in Example 1 | 0.005 |
| Flavor | 1.0 |
| Water | 37.0 |
| Total | 100.0 |

Using each component having the above amount, dentifrice was prepared by a common method.

| Example 3 (gargle) | |
|---|---|
| Glycerin | 7.0 |
| Sorbitol | 5.0 |
| Ethyl alcohol | 15.0 |
| Sodium lauryl sulfate | 0.8 |
| Sodium saccharin | 0.09 |
| 1-menthol | 0.05 |
| Flavor | 0.045 |
| Hop bract polyphenol obtained as in Example 1 | 0.005 |
| Water | 72.0 |
| Total | 100.0 |

Using each component having the above amount, gargle was prepared by a common method.

| Example 4 (troche) | |
|---|---|
| Gum arabic | 6.0 |
| Magnesium stearate | 3.0 |
| Glucose | 73.0 |
| Lactose | 17.6 |
| Dipotassium phosphate | 0.2 |
| Potassium phosphate | 0.1 |
| Flavor | 0.095 |
| Hop bract polyphenol obtained as in Example 1 | 0.005 |
| Total | 100.0 |

Using each component having the above amount, troche was prepared by a common method.

| Example 5 (wheat gluten) | |
|---|---|
| Sucrose | 20.0 |
| Thick malt syrup (75% solid) | 70.0 |
| Water | 9.5 |
| Coloring agent | 0.45 |
| Flavor | 0.045 |
| Hop bract polyphenol obtained as in Example 1 | 0.005 |
| Total | 100.0 |

Using each component having the above amount, wheat gluten was prepared by a common method.

| Example 6 (Chewing gum) | |
|---|---|
| Gum base | 20.0 |
| Calcium carbonate | 2.0 |
| Lactose | 77.0 |
| Stevioside | 0.095 |
| Hop bract polyphenol obtained as in Example 1 | 0.005 |
| Flavor | 0.9 |
| Total | 100.0 |

Using each component having the above amount, chewing gum was prepared by a common method.

| Example 7 (juice) | |
|---|---|
| Concentrated orange juice | 15.0 |
| Fructose | 5.0 |
| Citric acid | 0.2 |
| Flavor | 0.1 |
| Coloring agent | 0.15 |
| Sodium ascorbate | 0.048 |
| Hop bract polyphenol obtained as in Example 1 | 0.005 |
| Water | 79.5 |
| Total | 100.0 |

Using each component having the above amount, juice was prepared by a common method.

| Example 8 (cookies) | |
|---|---|
| Soft flour | 32.0 |
| Whole egg | 16.0 |
| Butter | 16.0 |
| Sugar | 25.0 |
| Water | 10.8 |
| Baking powder | 0.198 |
| Hop bract polyphenol obtained as in Example 1 | 0.005 |
| Total | 100.0 |

Using each component having the above amount, cookies were prepared by a common method.

| Example 9 (Caramels) | |
|---|---|
| Granulated sugar | 31.0 |
| Thick malt syrup (75% solid) | 20.0 |
| Powder milk | 40.0 |
| Hardened oil | 5.0 |
| Sodium chloride | 0.6 |
| Flavor | 0.025 |
| Hop bract polyphenol obtained as in Example 1 | 0.005 |
| Water | 3.37 |
| Total | 100.0 |

Using each component having the above amount, caramels were prepared by a common method.

| Example 10 (tablet, capsule) | |
|---|---|
| Hop bract polyphenol obtained as in Example 1 | 10.0 |
| Lactose | 75.0 |
| Magnesium stearate | 15.0 |
| Total | 100.0 |

Using each component having the above amount, tablets and capsules were prepared by a common method.

| Example 11 (powdered drug, granule) | |
|---|---|
| Hop bract polyphenol obtained as in Example 1 | 20.0 |
| Starch | 30.0 |
| Lactose | 50.0 |
| Total | 100.0 |

Using a mixture of each component having the above amount, powdered drug and granule were prepared by a common method.

| Example 12 (injection agent) | |
|---|---|
| Hop bract polyphenol obtained as in Example 1 | 1.0 |
| Surfactant | 9.0 |
| Physiological saline | 90.0 |
| Total | 100.0 |

Using each component having the above amount, an injection agent was obtained by heat mixing and sterilization.

Example 13 (Determination of polyphenol weight in the hop bract polyphenol)

As to the hop bract polyphenol obtained in Example 1 and Comparison example 1, referencing the method of Folin et al (O. Folin and W. Denis, J. Biol. Chem. 22, 305-308 (1915), the content of polyphenol was determined. Concretely, the sample solution (an aqueous 20% methanol solution) 0.2 ml, distilled water 0.8 ml, a phenol reagent (manufactured by Kanto Kagaku Co.) 1.0 ml and an aqueous solution 5.0 ml of 0.4 M sodium carbonate were mixed. The mixture was left for 30 minutes at room temperature and the absorbance at 760 nm was determined with a spectrophotometer (Hitachi U-2000). As a standard sample, chlorogenic acid of a common natural polyphenol is used, and content per weight of chlorogenic acid is showed. Table 1 shows the results of determination of polyphenol amounts of Example 1 and Comparison example 1. From the results, polyphenol amount of Example 1 was more than that of Comparison example 1. It shows that the purity of polyphenol of Example 1 is higher than that of Comparison example 1.

Table 1

| Sample | Polyphenol content (%) |
|---|---|
| Example 1 | 83.7% |
| Comparison example 1 | 77.2% |

Example 14 (Inhibition effect for insoluble glucan-producing enzyme of dental caries bacteria)

As to the hop bract polyphenol obtained in Example 1 and Comparison example 1, referring the Nakahara et al's method (K. Nakahara, S. Kawabata, H. Ono, K. Ogura, T. Tanaka, T. Oshima and S. Hamada, Appl. Environ. Microbiol., 59, 968-973 (1993), the inhibition effect for insoluble glucan-producing enzyme of dental caries bacteria was studied. Typical dental caries, S. sobrinus (ATCC conserved strain 33478) was cultured and separated by centrifugation into fungus bodies and culture supernatant. This culture supernatant was salted out with a 50% ammonium sulfate solution, and the precipitate was dialyzed to obtain crude liquid of insoluble glucan-producing enzyme. The enzyme crude liquid, a test sample (final concentration 200μg/ml) and sucrose (final concentration 50mM) were mixed to obtain a total volume 750μl of a 100mM potassium phosphate buffer solution (pH6.5), and the solution was incubated at a temperature of 30°C for 16 hours. After the incubation, the mixed solution was centrifuged at 15,000 rpm for 10 minutes. After removing the supernatant, the precipitate was washed three times with 1:1 mixture of a buffer solution of 100 mM potassium phosphate (pH 6.5) and ethanol. The precipitate was dissolved into an aqueous solution of 1 N sodium hydroxide, and the glucogenesis was determined by a phenol sulfate method.

Fig. 1 shows the inhibition rates in Example 1 and Comparison example 1. From the results, it is found that inhibition activity for insoluble glucan-producing enzyme in Example 1 was higher than that in Comparison example 1. It shows that useful hop bract polyphenol having high functionality can be obtained by the method of this invention.

Industrial Applicability

Hop bract is used as the starting material, hop bract polyphenol having higher purity than that of conventional hop bract polyphenol can be obtained by this invention. The resulting hop bract polyphenol can be easily used as foods, drinks, cosmetics and drugs.

**Claims**

1.  A process for producing hop bract polyphenol comprising extracting hop bract with an aqueous alcohol solution, concentrating the extract to give a residual alcohol concentration of 2 v/v% or less, and purifying the concentrate.

2.  An eating and drinking product containing the hop bract polyphenol produced by the method of claim 1.

3.  A cosmetic containing the hop bract polyphenol produced by the method of claim 1.

4.  A drug containing the hop bract polyphenol produced by the method of claim 1.

5.  A drug as claimed in claim 4, it is selected from a group comprising an anti-inflammatory agent, an anti-allergy agent, an agent for inhibition of dental plaque formation, an anticancer agent, and an antimetastatic agent of cancer cells.

Fig.1

<div style="text-align:center">**INTERNATIONAL SEARCH REPORT**</div>

| International application No. |
|---|
| PCT/JP03/15959 |

**A. CLASSIFICATION OF SUBJECT MATTER**
Int.Cl$^7$   C07G17/00

According to International Patent Classification (IPC) or to both national classification and IPC

**B. FIELDS SEARCHED**

Minimum documentation searched (classification system followed by classification symbols)
Int.Cl$^7$   C07G17/00

Documentation searched other than minimum documentation to the extent that such documents are included in the fields searched

Electronic data base consulted during the international search (name of data base and, where practicable, search terms used)
CAPLUS/MEDLINE/BIOSIS/WPIDS(SNT)

**C. DOCUMENTS CONSIDERED TO BE RELEVANT**

| Category* | Citation of document, with indication, where appropriate, of the relevant passages | Relevant to claim No. |
|---|---|---|
| Y | JP 9-2917 A  (Asahi Breweries, Ltd.),<br>07 January, 1997 (07.01.97),<br>Claims; Par. Nos. [0011], [0033]; example 1<br>(Family: none) | 1-5 |
| Y | JP 9-295944 A  (Asahi Breweries, Ltd.),<br>18 November, 1997 (18.11.97),<br>Claims; Par. Nos. [0009], [0014]; examples 2,<br>7 to 18<br>(Family: none) | 1-5 |
| Y | JP 2001-39886 A  (Asahi Breweries, Ltd.),<br>13 February, 2001 (13.02.01),<br>Claims; examples 2, 9<br>(Family: none) | 1-5 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06 February, 2004 (06.02.04) | 17 February, 2004 (17.02.04) |

| Name and mailing address of the ISA/<br>Japanese Patent Office | Authorized officer |
|---|---|
| Facsimile No. | Telephone No. |

Form PCT/ISA/210 (second sheet) (July 1998)

**EP 1 577 315 A1**

<table>
<tr><td colspan="2" align="center">**INTERNATIONAL SEARCH REPORT**</td><td>International application No.<br>PCT/JP03/15959</td></tr>
</table>

| C (Continuation). | DOCUMENTS CONSIDERED TO BE RELEVANT | |
|---|---|---|
| Category* | Citation of document, with indication, where appropriate, of the relevant passages | Relevant to claim No. |
| Y | JP 2000-327582 A (Asahi Breweries, Ltd.),<br>28 November, 2000 (28.11.00),<br>Claims; examples 2, 4 12<br>(Family: none) | 1-5 |
| Y | JP 9-224606 A (Meiji Seika Kaisha, Ltd.),<br>02 September, 1997 (02.09.97),<br>Par. No. [0014]; example 1<br>(Family: none) | 1-5 |

Form PCT/ISA/210 (continuation of second sheet) (July 1998)